# EUROPEAN PATENT APPLICATION

(11) **EP 4 454 710 A1**
(43) Date of publication of application: **30.10.2024**
(21) Application number: 23382404.4
(22) Date of filing: 28.04.2023
(51) Int. Cl.: A61P 37/06, C07K 14/705

(54) **BISPECIFIC FUSION PROTEINS WITH IMMUNOSUPPRESSIVE ACTIVITY**

(71) Applicant: Universitat de Barcelona, 08028 Barcelona (ES); FUNDACIÓ HOSPITAL UNIVERSITARI VALL D'HEBRON INSTITUT DE RECERCA - VHIR, 08035 Barcelona (ES)
(72) Inventor: GRINYÓ BOIRA, Josep Maria, 08907 L'Hospitalet de Llobregat (ES); BESTARD MATAMOROS, Oriol, 08035 Barcelona (ES)
(74) Representative: Hoffmann Eitle

(57) **Abstract**

The invention provides a fusion polypeptide of formula (I), wherein: P1, located at the N-terminal end of the polypeptide, represents CTLA-4 or a CD80/CD86-binding fragment thereof; Fc, is an immunoglobulin Fc domain; L is a rigid peptide linker having an amino acid length from 10 to 50 amino acids, the rigid peptide linker being selected from the group selected from: an α-helix linker and a linker including one or more Pro residues; and P2 is PD-L2 or a PD1-binding fragment thereof.

The present invention also provides dimers, either heterodimers or homodimers, including the polypeptide of formula (I), as well as pharmaceutical compositions and uses thereof as immunosuppressive agents.

P1-Fc-L-P2 (I)

## Description

### Technical Field

This disclosure relates to the field of bispecific fusion proteins. In particular, the invention relates to fusion protein and dimers which are useful as immunosuppressive agents, being suitable in the treatment of autoimmune diseases or in the treatment or prevention of transplant rejection, among others.

### Background Art

The human immune system protects the body from damage by foreign substances and pathogens. One way in which the immune system protects the body is by producing specialized cells, referred to as T lymphocytes or T cells. Intercellular interactions between T cells and antigen-presenting cells (APCs) generate T cell stimulatory signals that in turn lead to T cell responses to antigens. Full T cell activation requires Signal 1, which consists on the binding of the T cell receptor (TCR) to antigen-MHC complex present on antigen-presenting cells, and Signal 2, based on the binding of the receptor CD28 on the surface of the T cell to the CD86 and/or CD80 ligands present on the APCs. Without this second signal, Signal 1 leads the T cell anergic, unable to secrete cytokines and undergoes apoptosis. Signal 3 is mediated by the binding of secreted interleukins, mainly IL-2, to their cell surface receptors, which leads to T cell proliferation and clonal expansion.

Due to the crucial role that costimulatory signals play in T cell mediated immune responses, costimulatory pathways have been targeted to induce immunosuppression in conditions where the immune system acts in a non-beneficial manner to the organism. In the last two decades, several costimulatory pathways have been targeted using monoclonal antibodies or proteins. In fact, some of the agents developed for the inhibition of the CD80/86-CD28 and CD40-CD40L pathways have already entered in the clinic for the treatment of human autoimmune diseases or for the prevention of solid organ transplantation rejection.

For instance, soluble forms of CTLA4- an inhibitory effector of the CD80/86 pathway- have been constructed by fusing the variable-like extracellular domain of CTLA4 to immunoglobulin constant domains to provide CTLA4-Fc fusion proteins. Soluble CTLA4- Fc has been shown to prevent CD28-dependent co-stimulation by binding to both CD86 and CD80, and to inhibit co-stimulation of T cells and have beneficial immunosuppression effects in humans (Bruce SP. et al. ,"Update on abatacept: a selective co-stimulation modulator for rheumatoid arthritis", Ann Pharmacother., 2007, vol. 41(7), pp. 1153-62). However, not all patients respond to CTLA4-Fc and continued response requires frequent drug administration, perhaps in part because blockade of interaction of CD28 with CD86/CD80 is a weak inducer of Tregs and insufficient for blocking activated effector T cell responses in a disease milieu.

The PD1-PD-L1/PD-L2 pathway is also under strong investigation for the development of immunosuppressive therapies. This costimulatory pathway consists of the programmed cell death-1 (PD-1) receptor and its ligands, PD-L1 and PD-L2, which deliver inhibitory signals that regulate the balance among T cell activation, tolerance, and immune- mediated tissue damage. Although most of the studies have been centered on the PD-L1 ligand, the higher affinity of PD-L2 and its expression pattern makes it a promising option for the development of immunomodulatory tools.

Attempts to improve the behaviour of both CTLA-4 and PD-L2 have been reported in the prior art. For example, the international application WO201004105 (hereinafter also referred as "WO2010'), among many fusion proteins, discloses a fusion protein comprising, in N- to C-terminal direction, CTLA-4 and PD-L2. However, it was found that the resulting fusion protein had a significantly lower binding capacity to PD-1 (K_{D} = 340 nM) than the moiety corresponding only to PD-L2 sequence bound to Fc (PD-L2-Fc, K_{D} = 26 nM). This prior art concluding that the C-terminal location of PD-L2 negatively affects to the binding to PD-1.

WO2020144332, directed to solve the limitations reported by WO2010', provides a PD-L2/CTLA-4 fusion protein with improved PD-1 activity with a design based on changing the order of the different portions, locating PD-L2 in the N-terminal region, instead of in the C-terminal.

On the other hand, tacrolimus, which is one of the immunosuppressive drugs of reference, has been widely reported as providing many side-effects as well as a heterogeneous response among the patients (cf. Peters DH et al., "Tacrolimus. A review of its pharmacology, and therapeutic potential in hepatic and renal transplantation", Drugs, 1993, 46(4):746-94).

In spite of the efforts made so far in the field of immunotherapy, therefore, there is still the need of molecules which provides with reliable and efficient modulation of the immunosuppressive pathways.

### Summary of Invention

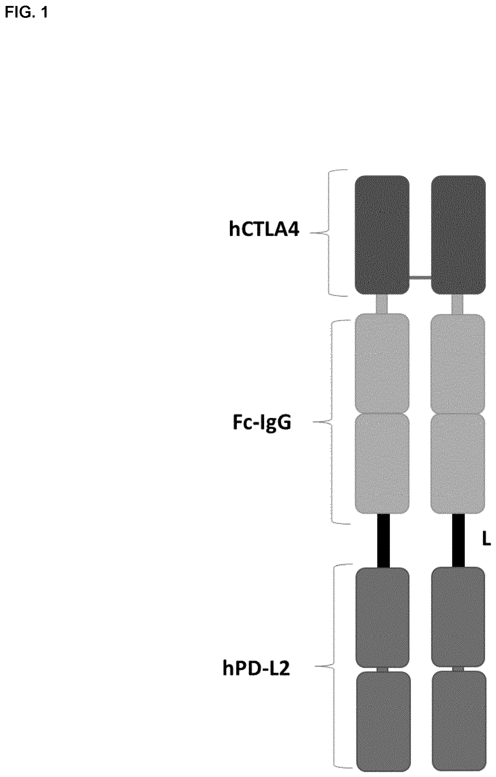

The inventors of the present invention have developed a dimer based on a fusion protein unit with a particular design. In particular, the dimer comprises two fusion protein units, each one of them comprising, in the N- to C-terminal direction, the following portions: CLTA-4, Fc, a rigid peptide linker (L), and PD-L2 (see FIG. 1).

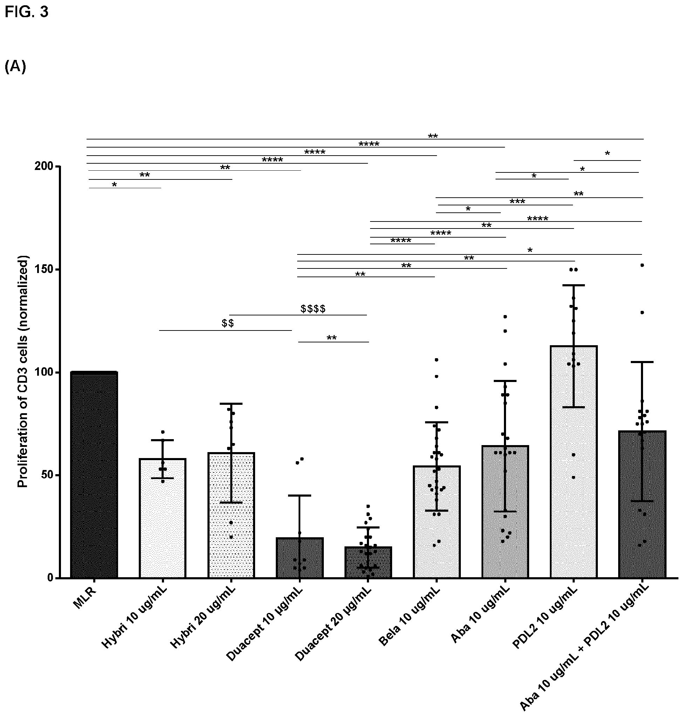

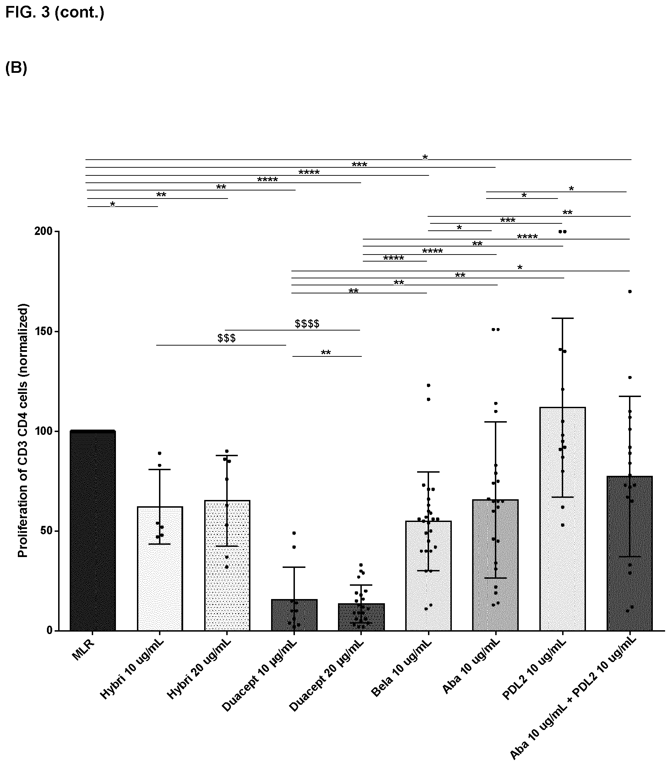

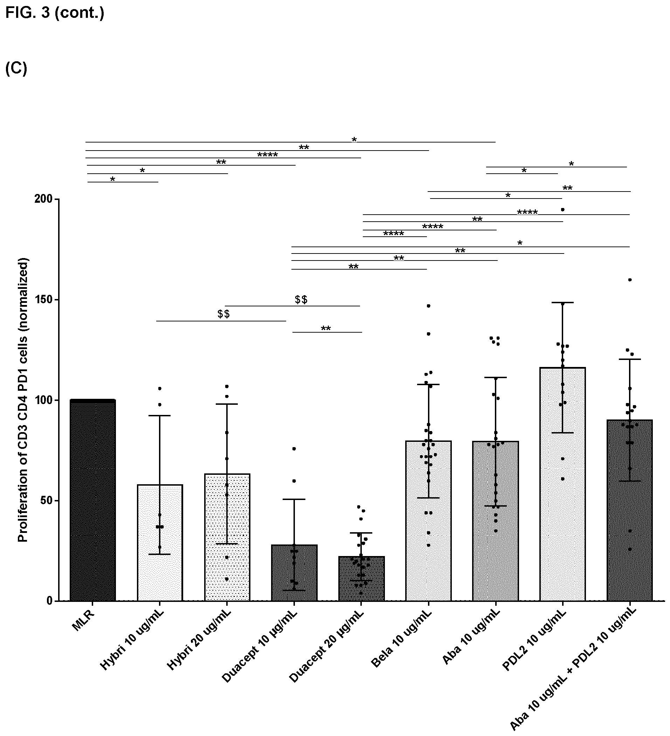

As it is shown below, this dimer was tested to confirm the possible impact on the co-stimulatory signal involved in the process of immune activation after transplantation. To that end, the effect on the *in vitro* proliferation of CD3⁺, CD3⁺CD4⁺ and CD3⁺CD4⁺PD-1⁺ T cells was determined. The effect provided by the dimer was compared with the effect provided by the dimer construct disclosed in WO2020144332 (hereinafter also referred as "HYBRI"), as well as with the separate administration of PD-L2+CTLA-4, and commercial treatments such as belatacept (fusion protein CTLA-4-Fc), abatacept (fusion protein CTLA-4-Fc), and PD-L2. The results are shown in FIG. 3.

Surprisingly, and contrary to the teachings of WO201004105, the construct of the invention, based on a fusion protein including the PD-L2 in C-terminal, was capable of remarkably improving the inhibition of proliferation of CD3⁺ T cells, CD3⁺ CD4⁺ cells (T helper cells) and TCD3⁺ CD4⁺ PD1⁺ (T helper cells expressing PD-1 molecule) in allogenic co-cultures (MLR) as compared to abatacept or belatacept alone, as well as to the combination of abatacept and PD-L2 given together, illustrating a higher effect of CTLA-4 on CD80 and CD86 (when compared with abatacept or belatacept), while assuring that PD-L2 moiety almost retained the innate affinity towards to PD-1 (when compared with PD-L2).

The synergistic inhibitory effect on the proliferation of CD3⁺ T cells, CD3⁺ CD4⁺ cells (T helper cells) and TCD3⁺ CD4⁺ PD1⁺ (T helper cells expressing PD-1 molecule) was found when compared with "HYBRI", wherein the PD-L2 is located on N-terminal and CTLA-4 fragment in C-terminal.

It has to be remarked that the improved effect reported with the construct of the invention is achieved at a substantial lower effective amount of CTLA-4 and PD-L2. When FIG. 3 shows the concentration used in each case, it is pointed out that 10 or 20 µg of the construct of the invention per mL of suspension are added. From this total amount of dimer, a portion substantially lower than 10 or 20 µg corresponds to CTLA-4 moiety (about 35% of the total weight of the construct) and to PD-L2 moiety (about 25% of the total weight of the construct) . And, even being used in such lower amount when compared to the administration of CTLA-4 or PD-L2 alone, the effect on inhibiting the T cell proliferation is synergistically reduced. In other words, the particular design provided in the present invention allows to improve the efficiency in inhibiting T cell proliferation using remarkably lower amounts of the active agents CTLA-4 and PD-L2.

Thus, in a first aspect the present invention provides a fusion polypeptide of formula (I):

P1-Fc-L-P2 (I)

wherein:
P1, located at the N-terminal end of the polypeptide, represents CTLA-4 or a CD80/CD86-binding fragment thereof,
Fc, is an immunoglobulin Fc domain,
L is a rigid peptide linker having an amino acid length from 10 to 50 amino acids, the rigid peptide linker being selected from the group consisting of: an α-helix linker and a linker including one or more Pro residues; and
P2 is PD-L2 or a PD1-binding fragment thereof.

The inventors have also found that the effect provided by the construct of the invention is of the same extent as the one provided by the treatment of reference, tacrolimus. Not only that, but also that the response, in the case of the administration of the construct of the invention, is more homogenous among the samples tested. The latter is of special relevance in the managing of the therapeutic dosage regimens.

Altogether the data provided herein allow to conclude that the construct of the invention is suitable as immunosuppressive agent.

In a second aspect the present invention provides a dimer comprising two subunits, wherein one or both subunits correspond to the fusion polypeptide as defined in the first aspect of the invention.

In a third aspect the present invention provides a polynucleotide which encodes the fusion polypeptide as defined in the first aspect of the invention or the dimer as defined in the second aspect of the invention.

In a fourth aspect the present invention provides a vector comprising the polynucleotide as defined in the third aspect of the invention.

In a fifth aspect the present invention provides a host cell which is transformed or transfected with the polynucleotide as defined in the third aspect of the invention or the vector as defined in the fourth aspect of the invention.

In a sixth aspect the present invention provides a cell culture comprising the host cell as defined in the fifth aspect of the invention.

In a seventh aspect the present invention provides a process for the production of a fusion polypeptide as defined in the first aspect of the invention, the method comprising: (a) culturing the host cell as defined in the fifth aspect of the invention under reducing conditions; or, alternatively, (b) *in vitro* transcription and/or translation of the polynucleotide as defined in the third aspect of the invention under reducing conditions; and (c) isolating the resulting polypeptide.

In an eighth aspect the present invention provides a process for the production of a dimer as defined in the second aspect of the invention, the method comprising: (a) culturing the host cell as defined in the fifth aspect of the invention under non-reducing conditions; or, alternatively, (b) *in vitro* transcription and/or translation of the polynucleotide as defined in the third aspect of the invention under non-reducing conditions; and, optionally, (c) purifying the resulting dimer.

In case of not performing the purification step, or depending on the particular purification step, the resulting product of the process of the eighth aspect of the invention will include the dimers, as such, as well as in the form of aggregates, due to electrostatic interactions between aminoacidic residues due to the reaction medium conditions.

Thus, in a ninth aspect the present invention provides a composition obtainable by the process as defined in the eighth aspect of the invention.

In a tenth aspect the invention provides a pharmaceutical composition comprising a therapeutically effective amount of the fusion polypeptide and/or the dimer, as defined in the present invention, with at least one pharmaceutically acceptable carrier or excipient.

In an eleventh aspect the present invention provides a kit of parts comprising:
- the fusion polypeptide, the dimer, or the pharmaceutical composition, as defined in the first aspect of the invention, and
- optionally, instructions for its use.

In a twelfth aspect the present invention provides the fusion polypeptide, dimer, or pharmaceutical composition, as defined in the present invention, for use in therapy or diagnosis.

In a thirteenth aspect the present invention provides a fusion polypeptide, dimer, or pharmaceutical composition as defined in the present invention, for use in a method for inhibiting T cell activation, particularly for use in the treatment of a disease selected from an autoimmune disease or transplant rejection. This aspect can also be formulated as the use of a fusion polypeptide, dimer, or pharmaceutical composition, as defined in the present invention, for the manufacture of a medicament for the treatment of a disease by inhibiting T cell activation, particularly for the treatment of a disease selected from an autoimmune disease or transplant rejection. This aspect can also be alternatively formulated as a method for inhibiting the T cell proliferation, particularly for the treatment of an autoimmune or graft rejection disease, the method comprising administering a therapeutically effective amount of the fusion protein, dimer or pharmaceutical composition, as defined in the present invention, to a subject in need thereof.

In a final aspect the present invention provides an *in vitro* use of the fusion polypeptide, dimer, or pharmaceutical composition, as defined in the present invention, in a method for inhibiting T cell activation in an isolated biological sample of a subject.

### Brief Description of Drawings

Fig. 1 corresponds to a representation of the dimeric construct comprising [CTLA-4]-[Fc-lgG]-[L]-[PD-L2].

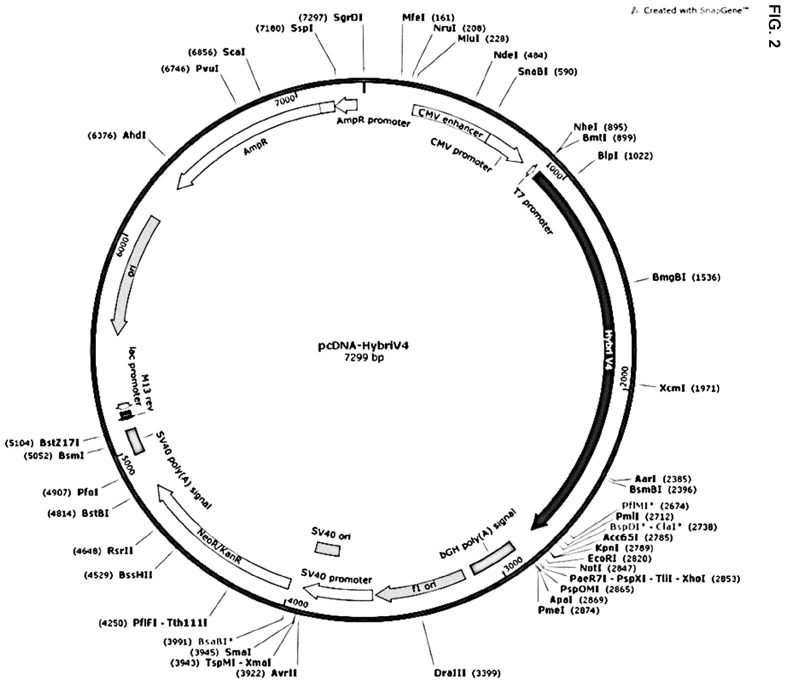

Fig. 2 corresponds to the map of the expression vector pcDNA3.1 used for expressing the fusion polypeptide in Example 1.
Fig. 3 shows the T cell proliferation (all results expressed as percentage of proliferation compared with the condition without drug (MLR)) in (A) CD3⁺ cells; (B) CD3⁺CD4⁺ cells; and (C) CD3⁺CD4⁺PD-1⁺ cells, under different treatments.

### Detailed Description of the Invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As mentioned above, the invention provides, in a first aspect, a fusion polypeptide of formula (I). In the context of the present invention "fusion polypeptide" and "fusion protein" have the same meaning and are used interchangeably.

The term "CTLA-4" as used herein refers to the protein named Cytotoxic T-lymphocyte protein 4, or CD152. It is formed by an extracellular domain Ig-like V-type domain, a transmembrane domain, and a cytoplasmic domain. The protein sequence from various species is available in several protein databases, such as Uniprot P16410_HUMAN Homo sapiens (10/01/2003 update); or P09793_MOUSE Mus musculus (01/07/1989 update). Human CTLA-4 is known to form homodimers through the cysteine 120 of its extracellular domain. A "CD80/86-binding fragment" of CTLA-4 refers to any fragment of a CTLA-4 protein capable of binding CD80/86. A way to test if a fragment maintains the ability of binding CD80/86 can be performed, for example, by mixed lymphocyte react (MLR) assays, as described in the examples bellow. Briefly, T cells from a subject are mixed with CD3⁺-depleted splenocytes and dendritic cells from another high HLA-mismatch subject, and the culture is treated either with the CD80/86-binding fragment or mocked treated. Proliferation is measured by quantifying the levels of CD3⁺ cells, and proliferations levels are compared between the group treated and mocked treated. If the level of proliferating CD3⁺ cells is significantly lower in the group treated with the fragment, this will be indicative that the fragment maintains the ability of binding to PD1.

In a particular embodiment of the first aspect, optionally in combination with any of the embodiments provided above and below, P1 is the extracellular domain of CTLA-4. In a more particular embodiment, CTLA-4 is mammalian CTLA-4, more particularly the extracellular domain of a mammalian CTLA-4. In a more particular embodiment, CTLA-4 is human CTLA-4, more particularly the extracellular domain of a human CTLA-4. In a more particular embodiment, the sequence of P1 has at least 85%, 86%, 87%, 88%, 89%, 90%, 91%, 92%, 93%, 94%, 95%, 96%, 97%, 98%, or 99% sequence identity with SEQ ID NO: 1. In a more particular embodiment, P1 is of sequence SEQ ID NO: 1.

The term "PD-L2" as used herein refers to the protein named programmed cell death 1 ligand 2, B7DC, CD273, or PDCD1 L2. It is formed by an extracellular domain, a transmembrane domain, and a cytoplasmic domain. The extracellular domain contains an Ig-like V-type domain and a Ig-like C2-type domain. The protein sequence from various species is available in several protein databases, such as Uniprot Q9BQ51_HUMAN Homo sapiens (10/05/2005 update); and Q9WUL5_MOUSE Mus musculus, (01/11/1999 update). A "PD1 -binding fragment" of PD-L2 refers to any fragment of a PD-L2 protein capable of binding PD1. A way to test if a fragment maintains the ability of binding PD1 can be performed, for example, by mixed lymphocyte react (MLR) assays, as described in the examples bellow. Briefly, T cells from a subject are mixed with CD3⁺-depleted splenocytes and dendritic cells from another high HLA-mismatch subject, and the culture is treated either with the candidate PD1 -binding fragment or mocked treated. Proliferation is measured by quantifying the levels of CD3⁺ cells, and proliferation levels are compared between the treated group and mocked treated group. If the levels of proliferating CD3⁺ cells are significantly lower in the group treated with the fragment, this will be indicative that the fragment maintains the ability of binding to PD1.

In a particular embodiment of the first aspect, optionally in combination with any of the embodiments provided above and below, P2 is the extracellular domain of PD-L2. In a more particular embodiment, PD-L2 is mammalian PD-L2, more particularly the extracellular domain of a mammalian PD-L2. In a more particular embodiment, PD-L2 is human PD-L2, more particularly the extracellular domain of a human PD-L2. In a more particular embodiment, the sequence of P2 has at least 85%, 86%, 87%, 88%, 89%, 90%, 91%, 92%, 93%, 94%, 95%, 96%, 97%, 98%, or 99% sequence identity with SEQ ID NO: 2. In a more particular embodiment, P2 is of sequence SEQ ID NO: 2.

In the present invention the term "identity" refers to the percentage of residues that are identical in the two sequences when the sequences are optimally aligned. If, in the optimal alignment, a position in a first sequence is occupied by the same amino acid residue as the corresponding position in the second sequence, the sequences exhibit identity with respect to that position. The level of identity between two sequences (or "percent sequence identity") is measured as a ratio of the number of identical positions shared by the sequences with respect to the size of the sequences (i.e., percent sequence identity = (number of identical positions/total number of positions) x 100). A number of mathematical algorithms for rapidly obtaining the optimal alignment and calculating identity between two or more sequences are known and incorporated into a number of available software programs. Examples of such programs include the MATCH BOX, MULTAIN, GCG, FASTA, and ROBUST programs for amino acid sequence analysis, among others. Preferred software analysis programs include the ALIGN, CLUSTAL W, and BLAST programs (e.g., BLAST 2.1, BL2SEQ, and later versions thereof).

For amino acid sequence analysis, a weight matrix, such as the BLOSUM matrixes (e.g., the BLOSUM45, BLOSUM50, BLOSUM62, and BLOSUM80 matrixes), Gonnet matrixes, or PAM matrixes (e.g., the PAM30, PAM70, PAM120, PAM160, PAM250, and PAM350 matrixes), are used in determining identity.

The BLAST programs provide analysis of at least two amino acid sequences, either by aligning a selected sequence against multiple sequences in a database (e.g., GenSeq), or, with BL2SEQ, between two selected sequences. BLAST programs are preferably modified by low complexity filtering programs such as the DUST or SEG programs, which are preferably integrated into the BLAST program operations. If gap existence costs (or gap scores) are used, the gap existence cost preferably is set between about -5 and -15. Similar gap parameters can be used with other programs as appropriate. The BLAST programs and principles underlying them are further described in, e.g., Altschul et al., "Basic local alignment search tool", 1990, J. Mol. Biol, v. 215, pages 403-410.

For multiple sequence analysis, the CLUSTAL W program can be used. The CLUSTAL W program desirably is run using "dynamic" (versus "fast") settings. Amino acid sequences are evaluated using a variable set of BLOSUM matrixes depending on the level of identity between the sequences. The CLUSTAL W program and underlying principles of operation are further described in, e.g., Higgins et al.,"CLUSTAL V: improved software for multiple sequence alignment", 1992, CABIOS, 8(2), pages 189-191.

As used herein, the "Fc region" contains the hinge region and the CH2 and CH3 constant domains of the heavy chain of an antibody. The term "hinge region" refers to the flexible region situated between the constant domains of the heavy chain CH1 and CH2.

Antibodies may be of any class, such as IgG, IgA, or IgM; and of any subclass, such as IgG1 or IgG4.

In a particular embodiment of the first aspect, optionally in combination with any of the embodiments provided above and below, the immunoglobulin Fc domain comprises the hinge region, the CH2 domain, and the CH3 domain of an immunoglobulin. In a more particular embodiment, the immunoglobulin is human IgG. In a particular embodiment, the Fc sequence has at least 85%, 86%, 87%, 88%, 89%, 90%, 91%, 92%, 93%, 94%, 95%, 96%, 97%, 98%, 99% or 100% sequence identity with SEQ ID NO: 3. In a more particular embodiment, the Fc is of sequence SEQ ID NO: 3.

The term "peptide linker" refers to an amino acid sequence which joins and separates two polypeptide domains in a protein. In the context of the invention, the linker is the sequence which joins the P1-Fc portion with the P2 domain of the polypeptide. The linker of the compound of the invention allows joining the N-terminal end of PD-L2 to the C-terminal end of Fc, without disrupting its binding ability to PD-1.

The terms "rigid peptide linker" and "rigid peptide" are used interchangeably.

In one embodiment, the rigid peptide linker adopts α-helix conformation or a salt-bridge-stabilized α-helix conformation.

As used herein, the term "α-helix" is a right-handed coiled conformation in which every backbone N-H group donates a hydrogen bond to the backbone C=O group of the amino acid four residues earlier (i+4→i hydrogen bonding). Amino acids that have a propensity for forming α-helical structure are typically methionine, alanine, leucine, glutamate, histidine, valine, tryptophan, glutamine, and lysine. In an embodiment, the amino acids having a propensity for forming an α-helical structure are selected from the group consisting of alanine, glutamate (glutamic acid), and leucine.

As used herein, the term "salt bridge-stabilized α-helix" refers to a peptide that has the propensity to form an α-helix wherein the peptide comprises oppositely charged residues every 1 or 4 residue apart (i+4) or 3 or 2 residues apart (i+3) (Marqusee, S. et al., "Proc. Natl. Acad. Sci., USA (1987) 84:8898-8902). In one embodiment, the rigid linker comprises a stabilized α-helix sequence of formula (II), (III) or (IV):

(Xaa1-Xaa1-Ala-Ala-Lys-Lys)_{d} (II)

(Xaa1-Ala-Ala-Ala-Xaa2)_{d} (III);

(Xaa1-Ala-Ala-Ala-Xaa2-Xaa3-Xaa3)_{d} (IV);

wherein: Xaa1=Glu or Asp; Xaa2=Lys or Arg; Xaa3=Leu, Ile, Val, Phe, Trp, Tyr, and Met; and d is an integer value from 2 to 10, particularly from 4 to 8.

In an alternative embodiment, the rigid linker comprises an extended proline dipeptide, which is particularly selected from sequences of formula (V), (VI), or (VII):

(Xaa4-Pro)ₑ (V);

(Pro-Xaa4)ₑ (VI);

and

[(Xaa4-Pro)₆-Gly-Gly]ₑ (VII)

wherein e is an integer value from 2 to 20; and Xaa4 is an acidic or a basic amino acid.

In one embodiment, the rigid peptide linker comprises at least 3 copies of the sequence of formula (III). Preferably the peptide linker molecule comprises from 4 to 8 copies of the motif of formula (III), with the length of the peptide linker molecule being extendible by the incremental addition of at least one amino acid.

In one embodiment, the rigid peptide linker comprises at least 3 copies of the sequence SEQ ID NO: 8:
SEQ ID NO: 8 EAAAK

In one embodiment, the construct also includes a flexible non-helical region which enables the domains to orientate into the binding sites of the target.

In a further embodiment of the invention the flexible non-helical region is located at or near the carboxyl-terminal end of the peptide linker molecule, thereby allowing the orientation of the binding domain located at the carboxyl-terminal end of the rigid peptide linker molecule in relation to its cognate receptor.

In a still further embodiment of the invention the flexible non-helical region is located at or near the amino and the carboxyl-terminal end of the peptide linker molecule, thereby allowing the orientation of the binding domains positioned at the amino and carboxyl-terminal end respectively of the peptide linker molecule in relation to their cognate receptor.

In another embodiment of the invention, there are no flexible connections between the rigid alpha helical linkers and the binding domains, and the PD-L2 moiety is bound to the Fc moiety by the rigid linker as defined above.

In different constructs the fixed orientation (both translational and rotational) provided by the peptide can be altered either by the insertion of 1, 2, 3, 4, 5, 6, 7, 8, 9 or 10 additional amino acids, or by the deletion of 1, 2, 3, 4, 5, 6, 7, 8, 9 or 10 amino acids to produce molecules with novel properties, for example antagonistic properties. Addition of an extra amino acid will produce an additional relative translation of the two domains by approximately 1.5 A and a relative rotation of the two domains around the helix axis of about +100°. Typically, linkers could start with two EAAAK units and will be lengthened by addition of A, AA, AAA, AAAA, EAAAA and EAAAK sequences. In one embodiment, the rigid linker of formula (II) to (IV) is extended by one or more alanine residues at the N- and C-terminal ends. In another embodiment, the rigid linker comprises at least 3, particularly from 4 to 8 copies of a peptide of (III), the rigid peptide further being inserted with one or more alanine residues both in the N-and C-terminal ends. In another embodiment, the rigid peptide linker comprises or consists of A(EAAAK)_{d}A, wherein d is an integer value in the range from 3 to 8, particularly d is 3, 4, 5, 6, 7, or 8. In one embodiment, the rigid peptide linker consists of A(EAAAK)₆A.

In a particular embodiment of the first aspect, optionally in combination with any of the embodiments provided above and below, the peptide linker is from 10 to 50 amino acids in length. In a more particular embodiment, the peptide linker is from 15 to 40 amino acids in length. In a more particular embodiment, the peptide linker is from 25 to 35 amino acids in length.

In the present invention, the term "amino acid" refers to a molecule containing both an amino group and a carboxyl group. Amino acids can be classified by the side chain group. There are basically four different classes of amino acids determined by different side chains: (1) non-polar, (2) polar and neutral, (3) acidic and polar, (4) basic and polar.

Non-polar amino acids have side chains which are hydrocarbon alkyl groups (alkane branches) or aromatic (benzene rings) or heteroaromatic (e.g. indole ring). Illustrative non- limitative examples of common non-polar amino acids are Ala, Val, Leu, lie, Pro, Trp, Gly, Phe, and Met.

Polar-neutral amino acids have polar but not charged groups at neutral pH in the side chain (such as hydroxyl, amide or thiol groups). Illustrative non-limitative examples of polar neutral amino acids are Ser, Thr, Cys, Tyr, Asn, and Gin.

Acid amino acids (hereinafter also referred as "acid and polar amino acid") have acidic side chains at neutral pH. These are aspartic acid or aspartate (Asp) and glutamic acid or glutamate (Glu), among others. Their side chains have carboxylic acid groups whose pKa's are low enough to lose protons, becoming negatively charged in the process.

Basic amino acids (hereinafter also referred as "basic and polar amino acid") have side chains containing nitrogen and resemble ammonia which is a base (such as amines, guanidines, or imidazole). Their pKa's are high enough that they tend to bind protons, gaining a positive charge in the process. Illustrative non-limitative examples of basic amino acids are Lys, Arg, and His.

The term "unnatural amino acid" comprises D-isomers of the 20 common naturally occurring alpha-amino acids or amino acids of formula (A) 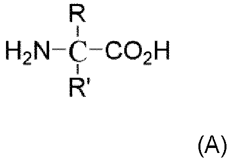 wherein R and R' have the meaning provided in Table 1 below. Further illustrative non-limitative examples of unnatural amino acids are summarized in Table 2:

**Table 1**

| Exemplary unnatural alpha-amino acids | Suitable amino acid side chains | |
|---|---|---|
| | R | R' |
| D-Alanine | -H | -CH₃ |
| D-Arginine | -H | -CH₂CH₂CH₂-NHC(=NH)NH₂ |
| D-Asparagine | -H | -CH₂C(=O)NH₂ |
| D-Aspartic acid | -H | -CH₂CO₂H |
| D-Cysteine | -H | -CH₂SH |
| D-Glutamic acid | -H | -CH₂CH₂CO₂H |
| D-Glutamine | -H | -CH₂CH₂C(=O)NH₂ |
| D-Histidine | -H | -CH₂-2-(1H-imidazole) |
| D-Isoleucine | -H | -sec-butyl |
| D-Leucine | -H | -iso-butyl |
| D-Lysine | -H | -CH₂CH₂CH₂CH₂NH₂ |
| D-Methionine | -H | -CH₂CH₂SCH₃ |
| D-Phenylalanine | -H | -CH₂Ph |
| D-Proline | -H | -2-(pyrrolidine) |
| D-Serine | -H | -CH₂OH |
| D-Threonine | -H | -CH₂CH(OH)(CH₃) |
| D-Tryptophan | -H | -CH₂-3-(1H-indole) |
| D-Tyrosine | -H | -CH₂-(p-hydroxyphenyl) |
| D-Valine | -H | -isopropyl |
| Di-vinyl | -CH=CH₂ | -CH=CH₂ |
| | | |

| Exemplary unnatural alpha-amino acids | R and R' are equal to: | |
|---|---|---|
| α-methyl-Alanine (Aib) | -CH₃ | CH₃ |
| α-methyl-Arginine | -CH₃ | -CH₂CH₂CH₂-NHC(=NH)NH₂ |
| α-methyl-Asparagine | -CH₃ | -CH₂C(=O)NH₂ |
| α-methyl-Aspartic | -CH₃ acid | -CH₂CO₂H |
| α-methyl-Cysteine | -CH₃ | -CH₂SH |
| α-methyl-Glutamic acid | -CH₃ | α-CH₂CH₂CO₂H |
| α-methyl-Glutamine | -CH₃ | -CH₂CH₂C(=O)NH₂ |
| α-methyl-Histidine | -CH₃ | -CH₂-2-(1H-imidazole) |
| α-methyl-Isoleucine | -CH₃ | -sec-butyl |
| α-methyl-Leucine | -CH₃ | -iso-butyl |
| α-methyl-Lysine | -CH₃ | -CH₂CH₂CH₂CH₂NH₂ |

**Table 2**

| | |
|---|---|
| Aad | 2-Aminoadipic acid |
| bAad | 3-Aminoadipic acid |
| bAla | beta-Alanine, beta-Aminopropionic acid |
| Abu | 2-Aminobutyric acid |
| 4Abu | 4-Aminobutyric acid, piperidinic acid |
| Acp | 6-Aminocaproic acid |
| Ahe | 2-Aminoheptanoic acid |
| Aib | 2-Aminoisobutyric acid |
| bAib | 3-Aminoisobutyric acid |
| Apm | 2-Aminopimelic acid |
| Dbu | 2,4 Diaminobutyric acid |
| Des | Desmosine |
| Dpm | 2,2'-Diaminopimelic acid |
| Dpr | 2,3-Diaminopropionic acid |
| EtGly | N-Ethylglycine |
| EtAsn | N-Ethylasparagine |
| Hyl | Hydroxylysine |
| aHyl | allo-Hydroxylysine |
| 3Hyp | 3-Hydroxyproline |
| 4Hyp | 4-Hydroxyproline |
| Ide | Isodesmosine |
| alle | allo-Isoleucine |
| Nva | Norvaline |
| Nle | Norleucine |
| Orn | Ornithine |

Each one of the amino acids forming the peptide of the invention can have, independently from the others, L- or D-configuration.

Amino acids used in the preparation of the polypeptides of the present invention may be prepared by organic synthesis, or obtained by other routes, such as, for example, degradation of or isolation from a natural source. In another embodiment of the first aspect of the invention, optionally in combination with any of the embodiments provided above or below, the compound consists of the fusion polypeptide of formula (I). In another embodiment of the first aspect of the invention, optionally in combination with any of the embodiments provided above or below, the compound consists of the fusion polypeptide of formula (I), and the N-terminal and C-terminal end corresponds to -NH₂ and -COOH, respectively. In another embodiment of the first aspect of the invention, optionally in combination with any of the embodiments provided above or below, the compound consists of the fusion polypeptide of formula (I), and the C-terminal and N-terminal are derivatized. It is well-known in the state of the art how to derivatize the terminal ends of a peptide. In one embodiment, optionally in combination with any of the embodiments provided above or below, the C-terminal is amidated (-C(O)NH₂). In one embodiment, optionally in combination with any of the embodiments provided above or below, the N-terminal is acetylated.

In another embodiment of the first aspect of the invention, optionally in combination with any of the embodiments provided above or below, the sequence of the fusion polypeptide has at least 85%, at least 90% or at least 95% identity with sequence SEQ ID NO: 11; preferably a 100% of identity with sequence SEQ ID NO: 11. In another embodiment, the fusion polypeptide consists of sequence SEQ ID NO: 11:
SEQ ID NO: 11

MHVAQPAVVLASSRGIASFVCEYASPGKATEVRVTVLRQADSQVTEVCAATYMMGNELTFL
DDSICTGTSSGNQVNLTIQGLRAMDTGLYICKVELMYPPPYYLGIGNGTQIYVIDPEPCPDSD
QEPKSSDKTHTSPPSPAPELLGGSSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPPVL
DSDGSFFLYSKLTVDKSRWQQGNVFSCSVMHEALHNHYTQKSLSLSPGKAEAAAKEAAAK
EAAAKEAAAKEAAAKEAAAKAFTVTVPKELYIIEHGSNVTLECNFDTGSHVNLGAITASLQKV
ENDTSPHRERATLLEEQLPLGKASFHIPQVQVRDEGQYQCIIIYGVAWDYKYLTLKVKASYR
KINTHILKVPETDEVELTCQATGYPLAEVSWPNVSVPANTSHSRTPEGLYQVTSVLRLKPPP
GRNFSCVFWNTHVRELTLASIDLQSQMEPRTHPT

As mentioned above, in a second aspect, the invention provides a dimer comprising two subunits, wherein one or both subunits correspond(s) to the fusion protein as defined in the first aspect.

All the embodiments of the fusion protein of the first aspect are meant to apply to the dimer of the second aspect.

In a particular embodiment of the second aspect, optionally in combination with any of the embodiments provided above or below, the dimer is a homodimer or a heterodimer. In a more particular embodiment, the subunits forming the dimer are linked to each other by at least one disulphide bond. In a more particular embodiment, the subunits forming the dimer are linked to each other by one disulphide bond. In an even more particular embodiment, the disulphide bond is located between the two P1 domains. In an even more particular embodiment, the dimer is a homodimer wherein P1 corresponds to the extracellular domain of CTLA-4 (SEQ ID NO: 1) and the disulphide bond is between the cysteine residue at position 120 of each one of the CTLA-4 extracellular domain. In an even more particular embodiment, the dimer is a homodimer wherein each one of the subunits comprises or consists of SEQ ID NO: 11, and the disulphide bond is between the cysteine residue at position 120 of each one of the CTLA-4 extracellular domain (SEQ ID NO: 1).

In a particular embodiment of the first or second aspects, optionally in combination with any of the embodiments provided above and below, the compound or the dimer further comprises a heterologous moiety.

As used herein, "heterologous moiety" refers to any molecule coupled to the fusion polypeptide via either a covalent or non-covalent bond. In a particular embodiment, the heterologous moiety is located in either the N-terminal or the C-terminal end of the compound. In a particular embodiment, the heterologous moiety is located in both the N- terminal and the C-terminal ends of the compound. The heterologous moiety can be, for example, a molecule that facilitates the purification of the protein. In a particular embodiment, the heterologous moiety is a peptide. In an even more particular embodiment, the heterologous moiety is a poly histidine track. In an even more particular embodiment, the heterologous moiety is a poly histidine track located in the C-terminal region of the compound or dimer. In a more particular embodiment, the poly histidine track consists of six histidines. As the skill in the art would understand, small peptides that assist in the purification of the protein can be maintained in the final compound without affecting its functionality.

Alternatively, the heterologous moiety can also be a diagnostic agent or a therapeutic agent.

In a more particular embodiment, the therapeutic agent is an immunosuppressive agent. A non-limiting list of immunosuppressive agents that can be used in the invention are methotrexate, dactinomycin, cyclosporin, 6-mercaptopurine, cyclophosphamide, mycophenolate, prednisolone, sirolimus, dexamethasone, rapamycin, FK506, mizoribine, azothioprine, tacrolimus, adalimumab, certolizumab, etanercept, golimumab, infliximab, belimumab, alefacept, abatacept, belatacept, tozcilizumab, and ustekinumab.

The immunosuppressive agent can be conjugated to the fusion polypeptide using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active asters (such as disuccinimidyl suberate), aldehydes (such as glutareldehyde), bis-azido compounds (such as bis (p-azidobenzoyl)hexanediamine), bis-diazonium derivatives (such as bis-(p-diazoniumbenzoyl)- ethylenediamine), diisocyanates (such as tolyene 2,6-diisocyanate), and bis-active fluorine compounds (such as 1 ,5-difluoro-2, 4-dinitrobenzene). In a more particular embodiment, the diagnostic agent is a label. The word "label" when used herein refers to a detectable compound or composition which is conjugated directly or indirectly to the fusion polypeptide so as to generate a "labelled" compound. The label may be detectable by itself (e.g. radioisotope labels or fluorescent labels) or, in the case of an enzymatic label, may catalyze chemical alteration of a substrate compound or composition which is detectable.

The label may be directly, attached or may be attached via a linker (such as Adipic Acid Dihyrazide (ADH). The label may be attached by chemical conjugation. Methods of conjugating labels to polypeptides are known in the art. For example, carbodiimide conjugation may be used to conjugate labels to antibodies. Other methods for conjugating a label to an antibody can also be used. For example, sodium periodate oxidation followed by reductive alkylation of appropriate reactants can be used, as can glutaraldehyde cross- linking.

The heterologous moiety can also be any vehiculation agent to facilitate the absorption, transport and delivery of the compound or dimer of the invention.

As mentioned above, in a third aspect, the invention provides a polynucleotide which encodes the fusion polypeptide as defined in the first aspect. In a particular embodiment of the third aspect, optionally in combination with any of the embodiments provided above and below, the polynucleotide is DNA or RNA. In one embodiment, the polynucleotide sequence is at least 85%, 86%, 87%, 88%, 89%, 90%, 91%, 92%, 93%, 94%, 95%, 96%, 97%, 98%, 99% or 100% identical to sequence SEQ ID NO: 12. In another embodiment, the polynucleotide consists of SEQ ID NO: 12.

As mentioned before, in a fourth aspect, the invention provides a vector comprising the polynucleotide of the third aspect. Examples of suitable vectors and techniques include those conventionally used in biomedicine and known to the skilled person. In one embodiment, the vector is at least 85%, 86%, 87%, 88%, 89%, 90%, 91%, 92%, 93%, 94%, 95%, 96%, 97%, 98%, 99% or 100% identical to sequence SEQ ID NO: 13. In another embodiment, the polynucleotide consists of SEQ ID NO: 13.

As mentioned above, in a fifth aspect, the invention provides a host cell which is transformed or transfected with the polynucleotide or the vector of the invention. The skilled person would know which host cells are suitable for the synthesis of the protein of the invention.

In a particular embodiment of the fifth aspect, optionally in combination with any of the embodiments provided above and below, the host cell is a eukaryotic host cell. In a more particular embodiment, the eukaryotic cell is selected from the group consisting of a CHO, HEK293 or *Pichia pastoris* cell. In an even more particular embodiment, the eukaryotic cell is an ExpiCHO cell.

As mentioned before, in a sixth aspect the invention provides a cell culture comprising the host cell of the fifth aspect. Examples of suitable cell culture mediums and conditions include those conventionally used in cell biology and known to the skilled person.

As above mentioned, in a seventh aspect the invention provides process for the production of the fusion protein according to the first aspect. The skilled person is familiar with several standard methods to isolate the resulting compound from the cell culture or after in vitro transcription and/or translation, for instance, Protein A purification column purification.

In the context of the invention, "reducing conditions" are those that do not allow the formation of disulphide bonds between polypeptides, such as the conditions provided by buffers that contain beta-mercaptoethanol or dithiothreitol (DTT). Therefore, in a particular embodiment, the monomer can be obtained in a buffer comprising 1mM DTT. Thus, the compound of the first aspect of the invention is a monomer in reducing conditions, and spontaneously dimerize in non-reducing conditions through the disulphide bonds located in the R2 domain.

As above mentioned, an eighth aspect of the composition provides a process for the production of a dimer as defined in the second aspect of the invention comprising (a) culturing the host cell as defined in the fifth aspect; or (b) in vitro transcription and/or translation of the polynucleotide as defined in the third aspect; and (c) isolating the compound in non-reducing conditions. As used herein, "non-reducing conditions" refers to any condition that allows the formation of disulphide bonds between polypeptides or proteins. In a particular embodiment, the dimer is formed in an isotonic buffer with a pH similar to the isoelectric point of the fusion polypeptide, around pH 6.5-7.5. More in particular, the dimer is formed in a PBS buffer comprising 50 mM sodium phosphate, 150 mM NaCl, at pH 7.

As mentioned before, in a tenth aspect the invention provides a pharmaceutical composition comprising the fusion protein as defined in the first aspect of the invention, the dimer as defined in the second aspect of the invention (purified or mixed with aggregates of the dimer, depending on the process of preparation), with at least one pharmaceutically acceptable excipient, diluent or carrier.

The expression "pharmaceutical composition" encompasses both compositions intended for human as well as for non-human animals (i.e. veterinarian compositions).

The expression "pharmaceutically acceptable carriers or excipient" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and non-human animals without excessive toxicity, irritation, allergic response, immunogenicity or other problems or complications commensurate with a reasonable benefit/risk ratio.

Examples of suitable pharmaceutically acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

The relative amounts of the compound or the dimer, the pharmaceutically acceptable excipients, and/or any additional ingredients in a pharmaceutical composition of the invention will vary, depending upon the identity, size, and/or condition of the subject treated and further depending upon the route by which the composition is to be administered.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Excipients such as coloring agents, coating agents, sweetening, and flavoring agents can be present in the composition, according to the judgment of the formulator.

The pharmaceutical compositions containing the compound or the dimer of the invention can be presented in any dosage form, for example, solid or liquid, and can be administered by any suitable route, for example, oral, parenteral, topical, intranasal or sublingual route, for which they will include the pharmaceutically acceptable excipients necessary for the formulation of the desired dosage form, for example, topical formulations (ointment, creams, lipogel, hydrogel, etc.), eye drops, aerosol sprays, injectable solutions, osmotic pumps, etc.

Exemplary diluents include, but are not limited to, calcium carbonate, sodium carbonate, calcium phosphate, dicalcium phosphate, calcium sulphate, calcium hydrogen phosphate, sodium phosphate lactose, sucrose, cellulose, microcrystalline cellulose, kaolin, mannitol, sorbitol, inositol, sodium chloride, dry starch, corn-starch, powdered sugar, and combinations thereof. Exemplary granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation- exchange resins, calcium carbonate, silicates, sodium carbonate, cross-linked polyvinylpyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (Veegum), sodium lauryl sulfate, quaternary ammonium compounds, and combinations thereof.

Exemplary binding excipients include, but are not limited to, starch (e.g., corn-starch and starch paste); gelatin; sugars (e.g., sucrose, glucose, dextrose, dextrin, molasses, lactose, lactitol, mannitol); natural and synthetic gums (e.g., acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, polyvinylpyrrolidone), magnesium aluminium silicate (Veegum), and larch arabogalactan); alginates; polyethylene oxide; polyethylene glycol; inorganic calcium salts; silicic acid; polymethacrylates; waxes; water; alcohol; and combinations thereof.

Exemplary preservatives may include antioxidants, chelating agents, antimicrobial preservatives, antifungal preservatives, alcohol preservatives, acidic preservatives, and other preservatives. Exemplary antioxidants include, but are not limited to, alpha tocopherol, ascorbic acid, ascorbyl palmitate, ascorbyl stearate, ascorbyl oleate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate.

Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminium hydroxide, alginic acid, pyrogen-free water, isotonic saline, Ringer's solution, ethyl alcohol, and combinations thereof.

Exemplary lubricating agents include, but are not limited to, magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulphate, sodium lauryl sulphate, and combinations thereof.

As above mentioned, in a twelfth eleventh aspect the invention provides the compound, the dimer, or the pharmaceutical composition of the invention for use in therapy or diagnosis.

In a particular embodiment of the thirteenth aspect, optionally in combination with any of the embodiments above or below, the compound, the dimer, or the pharmaceutical composition is for the treatment and/or prevention of a disease selected from an autoimmune disease and transplant rejection.

In a particular embodiment of the thirteenth aspect, optionally in combination with any of the embodiments above or below, the autoimmune disease is selected from type 1 diabetes, Systemic Lupus Erythematosus, Rheumatoid Arthritis, inflammatory bowel disease, ulcerative colitis, Crohn's disease, multiple sclerosis, scleroderma, pemphigus vulgaris, psoriasis, atopic dermatitis, celiac disease, Chronic Obstructive Lung disease, Hashimoto's thyroiditis, Graves' disease, Sjogren's syndrome, Guillain-Barre syndrome, Goodpasture's syndrome, Addison's disease, autoimmune necrotising vasculitis, (Wegener's granulomatosis), primary biliary sclerosis, sclerosing cholangitis, autoimmune hepatitis, polymyalgia rheumatica, Raynaud's phenomenon, temporal arteritis, giant cell arteritis, autoimmune hemolytic anemia, pernicious anemia, polyarteritis nodosa, Behget's disease, primary biliary cirrhosis, uveitis, myocarditis, rheumatic fever, ankylosing spondylitis, glomerulonephritis, sarcoidosis, dermatomyositis, myasthenia gravis, polymyositis, alopecia areata, and vitiligo.

The expression "therapeutically effective amount" as used herein, refers to the amount of the fusion protein or the dimer that, when administered, is sufficient to prevent development of, or alleviate to some extent, one or more of the symptoms of the disease or disorder which is addressed. The particular dose of agent administered according to this invention will of course be determined by the particular circumstances surrounding the case, including the compound or the dimer administered, the route of administration, the particular condition being treated, and the similar considerations.

In a particular embodiment of the thirteenth aspect, optionally in combination with any of the embodiments above or below, the transplant rejection is solid organ transplant rejection.

In a particular embodiment of the thirteenth aspect, optionally in combination with any of the embodiments above or below, the fusion protein, dimer, or pharmaceutical composition of the invention is administered in combination, either sequentially or simultaneously, with an immunesuppressant or modulator. A list of suitable immune suppressants or modulators has been provided above.

As mentioned above, in a final aspect, the invention provides an in vitro use of the fusion polypeptide as defined in the first aspect, the dimer as defined in the second aspect, or the pharmaceutical composition as defined in the tenth aspect, in a method for inhibiting T cell activation in an isolated biological sample. This aspect can alternatively be formulated as a method for inhibiting T cell activation comprising the step of contacting an isolated biological sample of a subject, the fusion protein as defined in the first aspect, the dimer as defined in the second aspect, or the pharmaceutical composition as defined in the tenth aspect of the invention.

As used herein, the term "isolated biological sample" refers to any sample that contains T cells and that it is obtained from any tissue or fluid of a subject.

As used herein, the term "T cell" refers to cells of the immune system that contain the T cell receptor on their surface. They include CD4-positive (CD4⁺) helper T cells and CD8- positive (CD8<+>) cytotoxic T cells, where the former relates to promoting immune response and the latter relates to excluding virus-infected cells and tumor cells."T cell activation" refers to the metabolic, morphological and functional changes that occur in a T cell that ensue upon antigen recognition. The activation is commonly accompanied by cell proliferation, cytokine secretion, differentiation into effector cells and memory cells.

The results herein provided demonstrate that the fusion protein or dimer of the invention can be useful in reducing the rejection risk to an organ to be transplanted in a recipient by inhibiting T-cell activation. With this aim, the fusion protein or dimer can be administered to the subject in the form of a pharmaceutical composition, as provided above but, in addition, or alternatively, the extracted organ, previous to the transplantation, can be subjected to a treatment with a sterile solution, comprising the compound or dimer of the invention.

In a particular embodiment of the twelfth and thirteenth aspects, optionally in combination with any of the embodiments above or below, the isolated biological sample is an organ or a tissue.

All the embodiments of the first aspect are also meant to apply to the other aspects provided by the present invention.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples

### Example 1: Production and purification of CTLA4-Fc-L-PDL2 fusion protein

### Design of fusion protein and gene synthesis

A fusion protein comprising human PD-L2 extracellular domain (SEQ ID NO: 2), human CTLA4 extracellular domain (SEQ ID NO: 1) and the Fc of human IgG was synthesized (SEQ ID NO: 3).

First the sequence of the protein of interest was designed (SEQ ID NO: 14). Amino acids 1 to 37 correspond to human CTLA4 signal peptide, 38 to 161 correspond to Fc region mature human CTLA4 Ig-like V-type domain. Amino acids 162 to 394 correspond to IgG Fc region. Amino acid 395-426 correspond to a A(EAAAK)₆A linker. Amino acid 427 to 626 correspond to human PDL2 Ig-like C2-type domain.

The precursor of the fusion protein (SEQ ID NO: 14) contains a CTLA4 signal peptide that is not present in the mature form of the protein (SEQ ID NO: 11).

The amino acid sequence was converted to DNA and synthetized by the company Genscript (SEQ ID NO: 12).

The gene of the fusion protein was cloned in pcDNA^{™}3.1(+) (ThermoFisher Scientific, V79020) expression vector for transfection and expression on CHO cells. The vector map of pcDNA3.1 is showed in Fig. 1 (SEQ ID NO: 13).

### Transfection and cell culture

A vial of EXPI CHO Cells Thermo Fisher Scientific was thawed to prepare the inoculum in 125 mL shake flasks. Then 100 mL in a 500 mL flask, (2 flasks), was seeded at following conditions: 37°C, 8% CO₂, 125 rpm a 3.5x106 cells/mL The cell culture before transfection reached a cell density of 6.0 × 10⁶ cells/mL and 100% viability.

Transfection of ExpiCHO cells was performed using the Expifectamine transfection kit (GIBCO), following instructions of the manufacturer, in two flasks with 100mL each of ExpiCHO expression medium. Briefly, transfection was carried out with a DNA amount of 100 µg and 320 µl of Expifectamine per flask. The cells were incubated for several days at 37 °C in an orbital shaker incubator until the viability drops below 50%. Cultures were harvested and cells were separated from supernatant by centrifugation. The supernatants were frozen at -20°C until the purification. The final volume of the pooled volume from both flasks was 145 mL.

The confirmation of the dimer production was performed by SDS PAGE, under reducing and non-reducing conditions:

### Protocol for non-reducing conditions (absence of reducing agent in the medium):

1. Prepare the gel: Cast the SDS-PAGE gel (Thermo Fisher) with 12% acrylamide.
2.1. Load the gel: Load 10 µL of protein sample and molecular weight markers (Broad Range MW markers, Thermo Scientific Ref. 26634) onto the gel wells.
3.2. Run the gel: Run the gel at a constant voltage of 200V until the molecular weight marker reaches the bottom of the gel.
4.3. Stain the gel: Stain the gel with Coomassie blue staining solution (Biorad) to visualize the protein bands.
5.4. Image and analyze the gel: visual inspection of the stained gel and analysis of the protein bands.

### Protocol for reducing conditions (presence of the reducing agent in the medium):

1. Add reducing agent to the sample: Add a 4 µL of reducing agent (NuPAGE Sample Reducing Agent), to 40 µL of the protein sample and incubate at room temperature for 10-15 minutes.
2. Prepare the gel: Cast the SDS-PAGE gel (Thermo Fisher) with 12% acrylamide. Add reducing agent to the running buffer at a final concentration of 5 mM.
3. Load the gel: Load 10 µL of the reduced protein sample and molecular weight marker (Broad Range MW markers, Thermo Scientific Ref. 26634) in the gel wells.
4. Run the gel: Run the gel at a constant voltage of 200V until the molecular weight marker reaches the bottom of the gel.
5. Stain the gel: Stain the gel with Coomassie blue staining solution to visualize the protein bands.
6. Image and analyze the gel: visual inspection of the stained gel and analysis of the protein bands.

The reducing agent helps to break disulfide bonds in proteins, resulting in the separation of subunits that may otherwise be covalently linked.

Comparing both SDS-PAGE gels it was observed that the molecular weight of the nonreduced protein corresponded to the double of the reduced protein, confirming the formation of the interchain disulfide bonds.

### Protein purification

Protein purification was performed using Protein A chromatography.

The culture supernatant was thawed and summited to the purification process described below.

Before the purification, the chromatograph and columns were sanitized with NaOH to completely remove the endotoxins. All the buffers and solutions were endotoxin-free, and the recipients used were either single-use endotoxin-free or treated at 200°C.

We loaded the column with the 145 mL of the thawed culture supernatant

| | |
|---|---|
| - Column: | Amsphere A3, 1ml CV |
| - Equilibration buffer: | PBS pH 7.4 |
| - Elution buffer: | 50 mM Sodium Citrate, 100 mM NaCl pH 3.5 |
| - Dialysis Buffer: | PBS pH 7.4 |

The elution peak fractions were pooled with a final volume of 2 ml, dialysed with PBS buffer. and stored at 4°C until dosed. The pooled fractions were analysed with Spectophotometer UV5 Nano, manufactured by Mettler Toledo, before and after dialysis. The protein concentration was determined with the formula: 280nm absorbance x 0.866 = mg/mL.

### Example 2: In vitro proliferation assays

Frozen PBMC were thawed in RPMI medium (Gibco, Thermofisher scientific, Waltham, Massachusetts, USA) with 20% fetal bovine serum (Cytiva, Little Chalfont, Buckinghamshire, United Kingdom). 200 000 PBMC from an individual, stained with CelltraceTM Violet Dye (Thermofisher scientific), were cocultured with 200 000 PBMC from another individual stained with CelltraceTM Farr Red Dye (Thermofisher Scientific) in RPMI medium supplemented with 10 % FBS and penicilline-streptomycine (Cultek, Madrid, Spain) for 7 days. Cells whether either cocultured without drug (condition MLR, n=30), or with Belatacept 10 µg/mL (n=26, BMS, Lawrenceville, NJ, USA), Abatacept 10 µg/mL (n= 22, BMS), rhPD-L2/Fc (n=14, R&D Systems, Minneapolis, MN, USA), the combination of Abatacept 10 µg/mL and rhPD-L2/Fc (n=22), HYBRI 10 or 20 µg/mL (prepared as disclosed in WO2020144332, Example 1, Bioingenium, Barcelona, Spain, n= 6 and 8 respectively) and the dimer of the invention (DUACEPT) 10 or 20 µg/mL (Bioingenium, n=10 and 22 respectively) (for each condition mean of duplicate is considered). Cells were cultured alone or with phytohaemagglutinin as negative and positive controls.

At day seven, PBMCs were retrieved and stained with Fixable viability Stain 520 (BD Biosciences, Franklin Lakes, New Jersey, USA), and then with mouse anti-human CD3 (BD), mouse anti-human CD4 (Biolegend, San Diego, California, USA) and mouse anti-human PD-1 (Biolegend) antibodies in Brilliant Stain Buffer (BD Bioscience).

Cells were analyzed using a LSRFortessaTM flow cytometer with DIVA (BD Biosciences) and FlowJo Softwares (Tree Star, Ashland, OR, USA). For each condition, we assessed the proportion of PBMC with low Violet or Farr Red dye staining among CD3⁺ cells, C3⁺CD4⁺ cells and CD3⁺CD4⁺PD-1⁺ cells and proceeded to normalization to the MLR without drug condition.

### Results

### In vitro T cell proliferation assays

Proliferation of CD3⁺ cells (all results expressed as percentage of proliferation compared with the condition without drug (MLR)) was19.35 +/- 14.78 % for Tacrolimus (n=26), 54.23 +/- 21.42 % for Belatacept, 64.09 +/- 31.70 % for Abatacept, 112.7 +/- 29.56 % for PD-L2, 71.17 +/-33.85 % for the combination of Abatacept and PD-L2, 57.83 +/- 9.218 % for HYBRI 10 µg/mL, 60.75 +/- 23.99 % for HYBRI 20 µg/mL, 19.30 +/- 20.71 % for "DUACEPT" 10 µg/mL and 14.86 +/- 9.882 for "DUACEPT" 20 µg/mL.

All drugs except PD-L2 induced a significant inhibition of CD3⁺ cells proliferation compared to control MLR (p<0.0001 for Tacrolimus, Belatacept, Abatacept and "DUACEPT" 20 µg/mL, p=0.005 for Abatacept + PD-L2, p=0.0313 for HYBRI 10 µg/mL, p=0.0078 for HYBRI 20 µg/m and p=0.002 for "DUACEPT" 10 µg/mL). Inhibition of CD3⁺ cells proliferation was more efficient with Tacrolimus than with Belatacept, Abatacept, Abatacept + PD-L2 (p<0.0001 for all comparisons). Inhibition of cell proliferation with "DUACEPT" 10 or 20 µg/mL was not significantly different from Tacrolimus (p=0.0801 and p=0.1629, respectively), and was significantly increased compared to Belatacept, Abatacept, Abatacept + PD-L2 and HYBRI (p<0.0001 for all comparisons with "DUACEPT" 20 µg/mL). "DUACEPT" 20 µg/mL inhibited cell proliferation more efficiently than 10 µg/mL (p=0.002).

Proliferation of CD3⁺CD4⁺ cells (all results expressed as percentage of proliferation compared with the condition without drug (MLR)) was 23.34 +/- 20.03 % for Tacrolimus, 54.85 +/- 24.79 % for Belatacept, 65.64 +/- 39.09 % for Abatacept 111.8 +/- 44.89 % for PD-L2, 77.33 +/- 40.14 % for the combination of Abatacept and PD-L2, 62.17 +/- 18.73 % for HYBRI 10 µg/mL, 65.25 +/- 22.70 % for HYBRI 20 µg/mL, 15.50 +/- 16.49 % for "DUACEPT" 10 µg/mL and 13.55 +/-9.501 for "DUACEPT" 20 µg/mL.

Similarly to CD3⁺ cells, all drugs except PD-L2 induced a significant inhibition of CD3⁺ CD4⁺ cells proliferation compared to control MLR (p<0.0001 for Tacrolimus, Belatacept and "DUACEPT" 20 µg/mL, p=0.0009 for Abatacept, p=0.0159 for Abatacept + PD-L2, p=0.0313 for HYBRI 10 ug/mL, p=0.0078 for HYBRI 20 µg/mL, p=0.002 for "DUACEPT" 10 µg/mL). Inhibition of CD3⁺ cells proliferation was more efficient with Tacrolimus than with Belatacept, Abatacept, Abatacept + PD-L2 (p<0.0001 for all comparisons). Inhibition of cell proliferation with "DUACEPT" 20 µg/mL was significantly better than with Tacrolimus (p=0.0392), and was also significantly increased compared to Belatacept, Abatacept, Abatacept + PD-L2 and HYBRI (p<0.0001 for all comparisons). "DUACEPT" 20 µg/mL inhibited cell proliferation more efficiently than 10 µg/mL (p=0.0039).

In the specific subset of CD3⁺CD4⁺PD-1⁺ cells, cell proliferation (all results expressed as percentage of proliferation compared with the condition without drug (MLR)) was 25.65 +/-20.39 % for Tacrolimus, 79.69 +/- 28.15 % for Belatacept, 79.45 +/- 31.91 % for Abatacept 116.2 +/- 32.35 % for PD-L2, 90.17 +/- 30.28 % for the combination of Abatacept and PD-L2, 58.00 +/- 34.56 % for HYBRI 10 µg/mL, 63.50 +/- 34.79 % for HYBRI 20 µg/mL, 28.00 +/-22.69 for "DUACEPT" 20 µg/mL and 22.14 +/- 11.80 for "DUACEPT" 20 µg/mL.

In this CD3⁺CD4⁺PD-1⁺ cells subset, all drugs except PD-L2 and the combination of Abatacept and PD-L2 induced a significant inhibition of CD3⁺CD4⁺ cells proliferation compared to control MLR (p<0.0001 for Tacrolimus and "DUACEPT" 20 µg/mL, p=0.0011 for Belatacept, p=0.0137 for Abatacept, p=0.0938 for HYBRI 10 µg/mL, p=0.0397 for HYBRI 20 µg/mL and p=0.002 for "DUACEPT" 10 µg/mL). Inhibition of cells proliferation was more efficient with Tacrolimus than with Belatacept, Abatacept, Abatacept+PD-L2 (p<0.0001 for all comparisons). Inhibition of cell proliferation with "DUACEPT" 10 or 20 µg/mL was not significantly different from Tacrolimus (p= 0.2324 and p=0.1842 respectively), and was significantly increased compared to Belatacept, Abatacept, Abatacept + PD-L2 and HYBRI (p<0.0001 for all comparisons with "DUACEPT" 20 µg/mL).

### Discussion and Conclusions

The construct was effective in reducing T cell proliferation in an allogenic cellular co-culture. First, the construct was tested against CD3⁺ T cells. The aim of this MLR assay was to evaluate the effectivity of the dimer to inhibit antigen-driven mature T cells proliferation. After that, the construct was also tested in CD3+CD4+ cells or helper cells as these cells play a crucial role in alloimmune and autoimmune responses after antigen presentation by antigen presenting cells. Finally, the construct was assessed in other T-cell subset population such as CD3⁺ CD4⁺ T cells expressing PD-1. In fact, CD3⁺CD4⁺PD1⁺ T cells is a target population of one of the subunits of the construct of the invention, PD-L2. Interestingly, although PD-L2 is less abundant than PD-L1, the affinity of PD-1 for PD-L2 is significantly much higher. The unexpected and potent effect of the construct in this MRL assay in all three T cell populations demonstrates the immunosuppressive properties of this recombinant fusion protein capable of inhibiting T cell-mediated responses after antigen recognition, which takes place after solid organ transplantation and in autoimmune disorders.

Comparing the data shown, in the three MRL assays, samples treated with tacrolimus exhibited an increased standard deviation in the allogenic response when compared with the construct administration. The increase in the standard deviation may be sign of a heterogeneous response to tacrolimus. This variability is greatly reduced with the construct treatment. See Table 3 below:

**Table 3**

| **Agent** | **Proliferation of CD3⁺ cells (% of proliferation compared with the condition without drug)** | **Proliferation of CD3⁺CD4⁺ cells (% of proliferation compared with the condition without drug)** | **Proliferation of CD3⁺CD4⁺PD-1⁺ cells (% of proliferation compared with the condition without drug)** |
|---|---|---|---|
| Tacrolimus (10 ng/mL) | 19.35 +/- 14.78 % | 23.34 +/- 20.03 % | 25.65 +/- 20.39 % |
| Dimer (20 µg/mL) | 14.86 +/- 9.882 % | 13.55 +/- 9.501 % | 22.14 +/- 11.80 % |

The reduced variability displayed by the construct may result in a better management of the disease, being as efficient as tacrolimus in terms of potency, but more homogenous in terms of response variability. These results together with the known toxicity profile of tacrolimus pose the construct as an attractive and alternative therapeutic option for autoimmune diseases and organ transplant rejection management.

**Table 4: sequences referred along the specification with the corresponding ID number**

| **SEQ ID NO:** | **Sequence** | **Description** |
|---|---|---|
| SEQ ID NO:1 | | Human CTLA4 Ig-like V-type domain |
| SEQ ID NO:2 | FTVTVPKELYIIEHGSNVTLECNFDTGSHVNLGAITASLQKVEN DTSPHRERATLLEEQLPLGKASFHIPQVQVRDEGQYQCIIIYGV AWDYKYLTLKVKASYRKINTHILKVPETDEVELTCQATGYPLAE VSWPNVSVPANTSHSRTPEGLYQVTSVLRLKPPPGRNFSCVF WNTHVRELTLASIDLQSQMEPRTHPT | Human PD-L2 Ig-like C2-type domain |
| SEQ ID NO:3 | QEPKSSDKTHTSPPSPAPELLGGSSVFLFPPKPKDTLMISRTP EVTCVVVDVSHEDPEVKFNWYVDGVEVHNAKTKPREEQYNS TYRVVSVLTVLHQDWLNGKEYKCKVSNKALPAPIEKTISKAKG QPREPQVYTLPPSRDELTKNQVSLTCLVKGFYPSDIAVEWES NGQPENNYKTTPPVLDSDGSFFLYSKLTVDKSRWQQGNVFS CSVMHEALHNHYTQKSLSLSPGK | Human IgG Fc region |
| SEQ ID NO:4 | (Xaa1-Xaa1-Ala-Ala-Lys-Lys)_{d} | Rigid linker formula (II) |
| SEQ ID NO:5 | (Xaa4-Pro)ₑ | Rigid linker formula (V) |
| SEQ ID NO:6 | (Pro-Xaa4)ₑ | Rigid linker formula (VI) |
| SEQ ID NO:7 | [(Xaa4-Pro)₆-Gly-Gly]ₑ | Rigid linker formula (VII) |
| SEQ ID NO:8 | EAAAK | Rigid linker |
| SEQ ID NO:9 | A(EAAAK)_{d}A | Rigid linker |
| SEQ ID NO:10 | AEAAAKEAAAKEAAAKEAAAKEAAAKEAAAKA | Rigid linker |
| SEQ ID NO:11 | MHVAQPAVVLASSRGIASFVCEYASPGKATEVRVTVLRQADS QVTEVCAATYMMGNELTFLDDSICTGTSSGNQVNLTIQGLRA MDTGLYICKVELMYPPPYYLGIGNGTQIYVIDPEPCPDSDQEP KSSDKTHTSPPSPAPELLGGSSVFLFPPKPKDTLMISRTPEVT | Mature fusion protein |
| | CVVVDVSHEDPEVKFNWYVDGVEVHNAKTKPREEQYNSTYR VVSVLTVLHQDWLNGKEYKCKVSNKALPAPIEKTISKAKGQPR EPQVYTLPPSRDELTKNQVSLTCLVKGFYPSDIAVEWESNGQ PENNYKTTPPVLDSDGSFFLYSKLTVDKSRWQQGNVFSCSV MHEALHNHYTQKSLSLSPGKAEAAAKEAAAKEAAAKEAAAKE AAAKEAAAKAFTVTVPKELYIIEHGSNVTLECNFDTGSHVNLGA ITASLQKVENDTSPHRERATLLEEQLPLGKASFHIPQVQVRDE GQYQCIIIYGVAWDYKYLTLKVKASYRKINTHILKVPETDEVELT CQATGYPLAEVSWPNVSVPANTSHSRTPEGLYQVTSVLRLKP PPGRNFSCVFWNTHVRELTLASIDLQSQMEPRTHPT | |
| SEQ ID NO:12 | ATGGCCTGCCTGGGCTTCCAGAGGCACAAGGCCCAGCTGA ACCTGGCTACCCGGACATGGCCTTGCACCCTGCTGTTCTTT CTGCTGTTCATCCCAGTGTTTTGTAAGGCTATGCATGTGGC TCAGCCAGCTGTGGTGCTGGCCTCCAGCAGGGGAATCGCC TCCTTCGTGTGCGAGTACGCCTCTCCCGGCAAGGCTACCG AGGTGAGAGTGACAGTGCTGAGGCAGGCTGACAGCCAGGT GACCGAGGTGTGCGCCGCTACATATATGATGGGCAACGAG CTGACCTTTCTGGACGATTCTATCTGTACCGGCACATCTTC CGGCAACCAAGTGAATCTGACCATCCAGGGCCTGCGGGCT ATGGATACAGGCCTGTACATCTGCAAGGTGGAGCTGATGTA TCCCCCTCCATACTATCTGGGCATCGGCAATGGCACCCAG ATCTACGTGATCGACCCAGAGCCCTGTCCTGACTCTGATCA GGAGCCTAAGAGCTCTGATAAGACCCACACATCCCCACCTA GCCCAGCTCCAGAGCTGCTGGGCGGCTCCAGCGTGTTCCT GTTTCCACCCAAGCCAAAGGACACCCTGATGATCAGCAGAA CCCCCGAGGTGACATGCGTGGTGGTGGACGTGTCTCACGA GGATCCAGAGGTGAAGTTTAACTGGTACGTGGATGGCGTG GAGGTGCATAATGCCAAGACAAAGCCCAGAGAGGAGCAGT ACAATTCTACCTATCGCGTGGTGTCCGTGCTGACAGTGCTG CATCAGGACTGGCTGAACGGCAAGGAGTATAAGTGCAAGG TGTCCAATAAGGCCCTGCCTGCTCCAATCGAGAAGACCATC AGCAAGGCCAAGGGCCAGCCCAGAGAGCCTCAGGTGTACA CACTGCCTCCATCCCGCGACGAGCTGACCAAGAACCAGGT GAGCCTGACATGTCTGGTGAAGGGCTTCTATCCTTCTGATA TCGCTGTGGAGTGGGAGTCCAATGGCCAGCCAGAGAACAA TTACAAGACCACACCCCCTGTGCTGGACTCTGATGGCTCCT | Nucleotide sequence of the construct |
| | TCTTTCTGTATTCCAAGCTGACCGTGGATAAGAGCCGCTGG CAGCAGGGCAACGTGTTTTCTTGCTCCGTGATGCACGAGG CCCTGCACAATCATTACACACAGAAGAGCCTGTCTCTGTCC CCTGGCAAGGCTGAGGCTGCTGCTAAGGAAGCCGCTGCTA AGGAGGCCGCTGCCAAGGAGGCTGCCGCTAAGGAGGCTG CTGCCAAAGAGGCTGCTGCTAAGGCCTTCACCGTGACAGT GCCAAAGGAGCTGTATATCATCGAGCACGGCTCTAACGTG ACCCTGGAGTGTAATTTTGACACAGGCTCCCATGTGAACCT GGGCGCCATCACCGCCTCCCTGCAGAAGGTGGAGAATGAT ACCTCTCCACACAGGGAGAGGGCCACACTGCTGGAGGAGC AGCTGCCACTGGGCAAGGCTTCCTTCCATATCCCTCAGGT GCAGGTGAGAGACGAGGGCCAGTACCAGTGCATCATCATC TATGGCGTGGCCTGGGATTACAAGTATCTGACCCTGAAGGT GAAGGCCTCCTACCGCAAGATCAACACCCACATCCTGAAG GTGCCCGAGACAGACGAGGTGGAGCTGACCTGTCAGGCC ACAGGCTATCCTCTGGCTGAGGTGAGCTGGCCTAACGTGT CTGTGCCAGCCAATACCAGCCATTCTAGAACACCTGAGGG CCTGTACCAGGTGACCTCTGTGCTGAGGCTGAAGCCACCA CCTGGAAGGAACTTCTCCTGCGTGTTCTGGAATACCCACGT GAGGGAGCTGACACTGGCTTCCATCGATCTGCAGAGCCAG ATGGAGCCACGGACCCATCCCACATGA | |
| SEQ ID NO:13 | GACGGATCGGGAGATCTCCCGATCCCCTATGGTGCACTCT CAGTACAATCTGCTCTGATGCCGCATAGTTAAGCCAGTATC TGCTCCCTGCTTGTGTGTTGGAGGTCGCTGAGTAGTGCGC GAGCAAAATTTAAGCTACAACAAGGCAAGGCTTGACCGACA ATTGCATGAAGAATCTGCTTAGGGTTAGGCGTTTTGCGCTG CTTCGCGATGTACGGGCCAGATATACGCGTTGACATTGATT ATTGACTAGTTATTAATAGTAATCAATTACGGGGTCATTAGT TCATAGCCCATATATGGAGTTCCGCGTTACATAACTTACGG TAAATGGCCCGCCTGGCTGACCGCCCAACGACCCCCGCCC ATTGACGTCAATAATGACGTATGTTCCCATAGTAACGCCAAT AGGGACTTTCCATTGACGTCAATGGGTGGAGTATTTACGGT AAACTGCCCACTTGGCAGTACATCAAGTGTATCATATGCCA AGTACGCCCCCTATTGACGTCAATGACGGTAAATGGCCCG CCTGGCATTATGCCCAGTACATGACCTTATGGGACTTTCCT ACTTGGCAGTACATCTACGTATTAGTCATCGCTATTACCATG | Nucleotide sequence of the plasmid |
| | GTGATGCGGTTTTGGCAGTACATCAATGGGCGTGGATAGC GGTTTGACTCACGGGGATTTCCAAGTCTCCACCCCATTGAC GTCAATGGGAGTTTGTTTTGGCACCAAAATCAACGGGACTT TCCAAAATGTCGTAACAACTCCGCCCCATTGACGCAAATGG GCGGTAGGCGTGTACGGTGGGAGGTCTATATAAGCAGAGC TCTCTGGCTAACTAGAGAACCCACTGCTTACTGGCTTATCG AAATTAATACGACTCACTATAGGGAGACCCAAGCTGGCTAG CATGGCCTGCCTGGGCTTCCAGAGGCACAAGGCCCAGCTG AACCTGGCTACCCGGACATGGCCTTGCACCCTGCTGTTCTT TCTGCTGTTCATCCCAGTGTTTTGTAAGGCTATGCATGTGG CTCAGCCAGCTGTGGTGCTGGCCTCCAGCAGGGGAATCGC CTCCTTCGTGTGCGAGTACGCCTCTCCCGGCAAGGCTACC GAGGTGAGAGTGACAGTGCTGAGGCAGGCTGACAGCCAG GTGACCGAGGTGTGCGCCGCTACATATATGATGGGCAACG AGCTGACCTTTCTGGACGATTCTATCTGTACCGGCACATCT TCCGGCAACCAAGTGAATCTGACCATCCAGGGCCTGCGGG CTATGGATACAGGCCTGTACATCTGCAAGGTGGAGCTGAT GTATCCCCCTCCATACTATCTGGGCATCGGCAATGGCACCC AGATCTACGTGATCGACCCAGAGCCCTGTCCTGACTCTGAT CAGGAGCCTAAGAGCTCTGATAAGACCCACACATCCCCAC CTAGCCCAGCTCCAGAGCTGCTGGGCGGCTCCAGCGTGTT CCTGTTTCCACCCAAGCCAAAGGACACCCTGATGATCAGCA GAACCCCCGAGGTGACATGCGTGGTGGTGGACGTGTCTCA CGAGGATCCAGAGGTGAAGTTTAACTGGTACGTGGATGGC GTGGAGGTGCATAATGCCAAGACAAAGCCCAGAGAGGAGC AGTACAATTCTACCTATCGCGTGGTGTCCGTGCTGACAGTG CTGCATCAGGACTGGCTGAACGGCAAGGAGTATAAGTGCA AGGTGTCCAATAAGGCCCTGCCTGCTCCAATCGAGAAGAC CATCAGCAAGGCCAAGGGCCAGCCCAGAGAGCCTCAGGT GTACACACTGCCTCCATCCCGCGACGAGCTGACCAAGAAC CAGGTGAGCCTGACATGTCTGGTGAAGGGCTTCTATCCTTC TGATATCGCTGTGGAGTGGGAGTCCAATGGCCAGCCAGAG AACAATTACAAGACCACACCCCCTGTGCTGGACTCTGATGG CTCCTTCTTTCTGTATTCCAAGCTGACCGTGGATAAGAGCC GCTGGCAGCAGGGCAACGTGTTTTCTTGCTCCGTGATGCA CGAGGCCCTGCACAATCATTACACACAGAAGAGCCTGTCTC | |
| | TGTCCCCTGGCAAGGCTGAGGCTGCTGCTAAGGAAGCCGC TGCTAAGGAGGCCGCTGCCAAGGAGGCTGCCGCTAAGGA GGCTGCTGCCAAAGAGGCTGCTGCTAAGGCCTTCACCGTG ACAGTGCCAAAGGAGCTGTATATCATCGAGCACGGCTCTAA CGTGACCCTGGAGTGTAATTTTGACACAGGCTCCCATGTGA ACCTGGGCGCCATCACCGCCTCCCTGCAGAAGGTGGAGAA TGATACCTCTCCACACAGGGAGAGGGCCACACTGCTGGAG GAGCAGCTGCCACTGGGCAAGGCTTCCTTCCATATCCCTC AGGTGCAGGTGAGAGACGAGGGCCAGTACCAGTGCATCAT CATCTATGGCGTGGCCTGGGATTACAAGTATCTGACCCTGA AGGTGAAGGCCTCCTACCGCAAGATCAACACCCACATCCT GAAGGTGCCCGAGACAGACGAGGTGGAGCTGACCTGTCA GGCCACAGGCTATCCTCTGGCTGAGGTGAGCTGGCCTAAC GTGTCTGTGCCAGCCAATACCAGCCATTCTAGAACACCTGA GGGCCTGTACCAGGTGACCTCTGTGCTGAGGCTGAAGCCA CCACCTGGAAGGAACTTCTCCTGCGTGTTCTGGAATACCCA CGTGAGGGAGCTGACACTGGCTTCCATCGATCTGCAGAGC CAGATGGAGCCACGGACCCATCCCACATGATAAGGTACCG AGCTCGGATCCACTAGTCCAGTGTGGTGGAATTCTGCAGAT ATCCAGCACAGTGGCGGCCGCTCGAGTCTAGAGGGCCCGT TTAAACCCGCTGATCAGCCTCGACTGTGCCTTCTAGTTGCC AGCCATCTGTTGTTTGCCCCTCCCCCGTGCCTTCCTTGACC CTGGAAGGTGCCACTCCCACTGTCCTTTCCTAATAAAATGA GGAAATTGCATCGCATTGTCTGAGTAGGTGTCATTCTATTCT GGGGGGTGGGGTGGGGCAGGACAGCAAGGGGGAGGATT GGGAAGACAATAGCAGGCATGCTGGGGATGCGGTGGGCT CTATGGCTTCTGAGGCGGAAAGAACCAGCTGGGGCTCTAG GGGGTATCCCCACGCGCCCTGTAGCGGCGCATTAAGCGC GGCGGGTGTGGTGGTTACGCGCAGCGTGACCGCTACACTT GCCAGCGCCCTAGCGCCCGCTCCTTTCGCTTTCTTCCCTTC CTTTCTCGCCACGTTCGCCGGCTTTCCCCGTCAAGCTCTAA ATCGGGGGCTCCCTTTAGGGTTCCGATTTAGTGCTTTACGG CACCTCGACCCCAAAAAACTTGATTAGGGTGATGGTTCACG TAGTGGGCCATCGCCCTGATAGACGGTTTTTCGCCCTTTGA CGTTGGAGTCCACGTTCTTTAATAGTGGACTCTTGTTCCAA ACTGGAACAACACTCAACCCTATCTCGGTCTATTCTTTTGAT | |
| | TTATAAGGGATTTTGCCGATTTCGGCCTATTGGTTAAAAAAT GAGCTGATTTAACAAAAATTTAACGCGAATTAATTCTGTGGA ATGTGTGTCAGTTAGGGTGTGGAAAGTCCCCAGGCTCCCC AGCAGGCAGAAGTATGCAAAGCATGCATCTCAATTAGTCAG CAACCAGGTGTGGAAAGTCCCCAGGCTCCCCAGCAGGCAG AAGTATGCAAAGCATGCATCTCAATTAGTCAGCAACCATAG TCCCGCCCCTAACTCCGCCCATCCCGCCCCTAACTCCGCC CAGTTCCGCCCATTCTCCGCCCCATGGCTGACTAATTTTTT TTATTTATGCAGAGGCCGAGGCCGCCTCTGCCTCTGAGCTA TTCCAGAAGTAGTGAGGAGGCTTTTTTGGAGGCCTAGGCTT TTGCAAAAAGCTCCCGGGAGCTTGTATATCCATTTTCGGAT CTGATCAAGAGACAGGATGAGGATCGTTTCGCATGATTGAA CAAGATGGATTGCACGCAGGTTCTCCGGCCGCTTGGGTGG AGAGGCTATTCGGCTATGACTGGGCACAACAGACAATCGG CTGCTCTGATGCCGCCGTGTTCCGGCTGTCAGCGCAGGGG CGCCCGGTTCTTTTTGTCAAGACCGACCTGTCCGGTGCCCT GAATGAACTGCAGGACGAGGCAGCGCGGCTATCGTGGCTG GCCACGACGGGCGTTCCTTGCGCAGCTGTGCTCGACGTTG TCACTGAAGCGGGAAGGGACTGGCTGCTATTGGGCGAAGT GCCGGGGCAGGATCTCCTGTCATCTCACCTTGCTCCTGCC GAGAAAGTATCCATCATGGCTGATGCAATGCGGCGGCTGC ATACGCTTGATCCGGCTACCTGCCCATTCGACCACCAAGC GAAACATCGCATCGAGCGAGCACGTACTCGGATGGAAGCC GGTCTTGTCGATCAGGATGATCTGGACGAAGAGCATCAGG GGCTCGCGCCAGCCGAACTGTTCGCCAGGCTCAAGGCGC GCATGCCCGACGGCGAGGATCTCGTCGTGACCCATGGCGA TGCCTGCTTGCCGAATATCATGGTGGAAAATGGCCGCTTTT CTGGATTCATCGACTGTGGCCGGCTGGGTGTGGCGGACCG CTATCAGGACATAGCGTTGGCTACCCGTGATATTGCTGAAG AGCTTGGCGGCGAATGGGCTGACCGCTTCCTCGTGCTTTA CGGTATCGCCGCTCCCGATTCGCAGCGCATCGCCTTCTAT CGCCTTCTTGACGAGTTCTTCTGAGCGGGACTCTGGGGTT CGAAATGACCGACCAAGCGACGCCCAACCTGCCATCACGA GATTTCGATTCCACCGCCGCCTTCTATGAAAGGTTGGGCTT CGGAATCGTTTTCCGGGACGCCGGCTGGATGATCCTCCAG CGCGGGGATCTCATGCTGGAGTTCTTCGCCCACCCCAACT | |
| | TGTTTATTGCAGCTTATAATGGTTACAAATAAAGCAATAGCA TCACAAATTTCACAAATAAAGCATTTTTTTCACTGCATTCTAG TTGTGGTTTGTCCAAACTCATCAATGTATCTTATCATGTCTG TATACCGTCGACCTCTAGCTAGAGCTTGGCGTAATCATGGT CATAGCTGTTTCCTGTGTGAAATTGTTATCCGCTCACAATTC CACACAACATACGAGCCGGAAGCATAAAGTGTAAAGCCTG GGGTGCCTAATGAGTGAGCTAACTCACATTAATTGCGTTGC GCTCACTGCCCGCTTTCCAGTCGGGAAACCTGTCGTGCCA GCTGCATTAATGAATCGGCCAACGCGCGGGGAGAGGCGGT TTGCGTATTGGGCGCTCTTCCGCTTCCTCGCTCACTGACTC GCTGCGCTCGGTCGTTCGGCTGCGGCGAGCGGTATCAGCT CACTCAAAGGCGGTAATACGGTTATCCACAGAATCAGGGG ATAACGCAGGAAAGAACATGTGAGCAAAAGGCCAGCAAAA GGCCAGGAACCGTAAAAAGGCCGCGTTGCTGGCGTTTTTC CATAGGCTCCGCCCCCCTGACGAGCATCACAAAAATCGAC GCTCAAGTCAGAGGTGGCGAAACCCGACAGGACTATAAAG ATACCAGGCGTTTCCCCCTGGAAGCTCCCTCGTGCGCTCT CCTGTTCCGACCCTGCCGCTTACCGGATACCTGTCCGCCTT TCTCCCTTCGGGAAGCGTGGCGCTTTCTCATAGCTCACGCT GTAGGTATCTCAGTTCGGTGTAGGTCGTTCGCTCCAAGCTG GGCTGTGTGCACGAACCCCCCGTTCAGCCCGACCGCTGCG CCTTATCCGGTAACTATCGTCTTGAGTCCAACCCGGTAAGA CACGACTTATCGCCACTGGCAGCAGCCACTGGTAACAGGA TTAGCAGAGCGAGGTATGTAGGCGGTGCTACAGAGTTCTT GAAGTGGTGGCCTAACTACGGCTACACTAGAAGAACAGTAT TTGGTATCTGCGCTCTGCTGAAGCCAGTTACCTTCGGAAAA AGAGTTGGTAGCTCTTGATCCGGCAAACAAACCACCGCTG GTAGCGGTGGTTTTTTTGTTTGCAAGCAGCAGATTACGCGC AGAAAAAAAGGATCTCAAGAAGATCCTTTGATCTTTTCTACG GGGTCTGACGCTCAGTGGAACGAAAACTCACGTTAAGGGA TTTTGGTCATGAGATTATCAAAAAGGATCTTCACCTAGATCC TTTTAAATTAAAAATGAAGTTTTAAATCAATCTAAAGTATATA TGAGTAAACTTGGTCTGACAGTTACCAATGCTTAATCAGTG AGGCACCTATCTCAGCGATCTGTCTATTTCGTTCATCCATA GTTGCCTGACTCCCCGTCGTGTAGATAACTACGATACGGGA GGGCTTACCATCTGGCCCCAGTGCTGCAATGATACCGCGA | |
| | GACCCACGCTCACCGGCTCCAGATTTATCAGCAATAAACCA GCCAGCCGGAAGGGCCGAGCGCAGAAGTGGTCCTGCAAC TTTATCCGCCTCCATCCAGTCTATTAATTGTTGCCGGGAAG CTAGAGTAAGTAGTTCGCCAGTTAATAGTTTGCGCAACGTT GTTGCCATTGCTACAGGCATCGTGGTGTCACGCTCGTCGTT TGGTATGGCTTCATTCAGCTCCGGTTCCCAACGATCAAGGC GAGTTACATGATCCCCCATGTTGTGCAAAAAAGCGGTTAGC TCCTTCGGTCCTCCGATCGTTGTCAGAAGTAAGTTGGCCGC AGTGTTATCACTCATGGTTATGGCAGCACTGCATAATTCTCT TACTGTCATGCCATCCGTAAGATGCTTTTCTGTGACTGGTG AGTACTCAACCAAGTCATTCTGAGAATAGTGTATGCGGCGA CCGAGTTGCTCTTGCCCGGCGTCAATACGGGATAATACCG CGCCACATAGCAGAACTTTAAAAGTGCTCATCATTGGAAAA CGTTCTTCGGGGCGAAAACTCTCAAGGATCTTACCGCTGTT GAGATCCAGTTCGATGTAACCCACTCGTGCACCCAACTGAT CTTCAGCATCTTTTACTTTCACCAGCGTTTCTGGGTGAGCA AAAACAGGAAGGCAAAATGCCGCAAAAAAGGGAATAAGGG CGACACGGAAATGTTGAATACTCATACTCTTCCTTTTTCAAT ATTATTGAAGCATTTATCAGGGTTATTGTCTCATGAGCGGAT ACATATTTGAATGTATTTAGAAAAATAAACAAATAGGGGTTC CGCGCACATTTCCCCGAAAAGTGCCACCTGACGTC | |
| SEQ ID NO:14 | MACLGFQRHKAQLNLATRTWPCTLLFFLLFIPVFCKAMHVAQP AVVLASSRGIASFVCEYASPGKATEVRVTVLRQADSQVTEVCA ATYMMGNELTFLDDSICTGTSSGNQVNLTIQGLRAMDTGLYIC KVELMYPPPYYLGIGNGTQIYVIDPEPCPDSDQEPKSSDKTHT SPPSPAPELLGGSSVFLFPPKPKDTLMISRTPEVTCVVVDVSH EDPEVKFNWYVDGVEVHNAKTKPREEQYNSTYRVVSVLTVLH QDWLNGKEYKCKVSNKALPAPIEKTISKAKGQPREPQVYTLPP SRDELTKNQVSLTCLVKGFYPSDIAVEWESNGQPENNYKTTP PVLDSDGSFFLYSKLTVDKSRWQQGNVFSCSVMHEALHNHY TQKSLSLSPGKAEAAAKEAAAKEAAAKEAAAKEAAAKEAAAKA FTVTVPKELYIIEHGSNVTLECNFDTGSHVNLGAITASLQKVEN DTSPHRERATLLEEQLPLGKASFHIPQVQVRDEGQYQCIIIYGV AWDYKYLTLKVKASYRKINTHILKVPETDEVELTCQATGYPLAE VSWPNVSVPANTSHSRTPEGLYQVTSVLRLKPPPGRNFSCVF WNTHVRELTLASIDLQSQMEPRTHPT | Immature fusion protein |

## Claims

1. A fusion polypeptide of formula (I):
P1-Fc-L-P2 (I)
wherein:
P1, located at the N-terminal end of the polypeptide, represents CTLA-4 or a CD80/CD86-binding fragment thereof,
Fc, is an immunoglobulin Fc domain,
L is a rigid peptide linker having an amino acid length from 10 to 50 amino acids, the rigid peptide linker being selected from the group selected from: an α-helix linker and a linker including one or more Pro residues; and
P2 is PD-L2 or a PD1-binding fragment thereof.

2. The fusion polypeptide according to claim 1, wherein the rigid peptide linker comprises a sequence selected from the group consisting of:
a) a salt bridge stabilized α-helix of formula (II) to (IV):
(Xaa1-Xaa1-Ala-Ala-Lys-Lys)_{d} (II)
(Xaa1-Ala-Ala-Ala-Xaa2)_{d}; (III)
(Xaa1-Ala-Ala-Ala-Xaa2-Xaa3-Xaa3)_{d}; (IV)
wherein: Xaa1=Glu or Asp; Xaa2=Lys or Arg; Xaa3=Leu, Ile, Val, Phe, Trp, Tyr, and Met; and d is an integer value from 2 to 10; and
b) an extended proline dipeptide of the formula (V) to (VII):
(Xaa4-Pro)ₑ (V)
(Pro-Xaa4)ₑ; (VI)
[(Xaa4-Pro)₆-Gly-Gly]ₑ (VII)
wherein e is an integer value from 2 to 20; and Xaa4 is an acidic or a basic amino acid.

3. The fusion polypeptide according to any of the claims 1-2, wherein the rigid linker is one of formula (II) to (VII) and it is extended by one or more alanine residues at the N- and C-terminal ends.

4. The fusion polypeptide according to any of the claims 1-3, wherein the rigid linker comprises at least 3, particularly from 4 to 8 copies of a peptide of formula (III); particularly it comprises or consists of the peptide of SEQ ID NO: 9; particularly it comprises or consists of SEQ ID NO: 10.

5. The fusion polypeptide according to any of the claims 1-4, wherein:
- P1 is the extracellular domain of CTLA-4, P2 is the extracellular domain of PD-L2, and the Fc region comprises the hinge region, the CH2 domain, and the CH3 domain of human IgG; or alternatively,
- the sequence of the fusion polypeptide has at least 85%, at least 90% or at least 95% identity with sequence SEQ ID NO: 11; preferably a 100% of identity with sequence SEQ ID NO: 11; preferably consists of SEQ ID NO: 11.

6. A dimer comprising two subunits, wherein one or both subunits comprise(s) the fusion polypeptide as defined in any of the claims 1-5.

7. The dimer according to claim 6, which comprises two fusion proteins of formula (I) linked by a disulphide bond between the two P1 fragments.

8. A polynucleotide which encodes the fusion polypeptide as defined in any of claims 1-7.

9. A vector comprising the polynucleotide as defined in claim 8.

10. A host cell which is transformed or transfected with the polynucleotide as defined in claim 8 or the vector as defined in claim 9.

11. A cell culture comprising the host cell as defined in claim 10.

12. A pharmaceutical composition comprising a therapeutically effective amount of the fusion polypeptide as defined in any of claims 1-5 and/or the dimer as defined in any of the claims 6-7, with at least one pharmaceutically acceptable carrier or excipient.

13. The fusion polypeptide as defined in any of claims 1-5, or the dimer as defined in any of the claims 6-7, or the pharmaceutical composition as defined in claim 12, for use in therapy or diagnosis.

14. The fusion polypeptide as defined in any of claims 1-5, or the dimer as defined in any of the claims 6-7, or the pharmaceutical composition as defined in claim 12 for use as immunosuppressive agent; particularly in a method for inhibiting T cell activation; more particularly for use in the treatment of a disease selected from an autoimmune disease and transplant rejection.

15. *In vitro* use of the fusion polypeptide as defined in any of claims 1-5, or the dimer as defined in any of the claims 6-7, or the pharmaceutical composition as defined in claim 12 in a method for inhibiting T cell activation in an isolated biological sample of a subject.
